(19) Europäisches Patentamt
European Patent Office
Office européen des brevets

(11) Publication number: **0 059 516**
**B1**

(12) **EUROPEAN PATENT SPECIFICATION**

(45) Date of publication of patent specification: **31.07.85**

(51) Int. Cl.$^4$: **C 07 C 126/067**, C 07 C 126/08

(21) Application number: **82200247.3**

(22) Date of filing: **27.02.82**

# (54) Process for the removal of urea, ammonia and carbon dioxide from dilute aqueous solutions.

(30) Priority: **28.02.81 NL 8100989**

(43) Date of publication of application:
**08.09.82 Bulletin 82/36**

(45) Publication of the grant of the patent:
**31.07.85 Bulletin 85/31**

(84) Designated Contracting States:
**AT BE DE FR GB IT NL SE**

(56) References cited:
**DE-A-2 721 462**
**FR-A-2 333 780**
**US-A-3 636 106**

**CHEMICAL ABSTRACTS, vol. 89, no. 22, 27th November 1978, page 105, no. 181820y, Columbus Ohio (USA);**
**CHEMICAL ENGINEERING PROCESS, vol. 73, no. 5, May 1977, pages 80-84, New York (USA); D.F. BRESS et al.: "The startup of two major *urea plants*".**

(73) Proprietor: **UNIE VAN KUNSTMESTFABRIEKEN B.V.**
**Maliebaan 81**
**NL-3581 CG Utrecht (NL)**

(72) Inventor: **van Nassau, Petrus Johannes Marie**
**Burg. Pijlsstraat 4**
**NL-6151 MA Sittard (NL)**
Inventor: **Douwes, Adolphe Marie**
**Potterstraat 34**
**NL-6165 AE Geleen (NL)**

(74) Representative: **Roeffen, Wilhelmus Johannes Maria et al**
**OCTROOIBUREAU DSM Postbus 9**
**NL-6160 MA Geleen (NL)**

**EP 0 059 516 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a process for the removal of urea, ammonia and carbon dioxide from dilute aqueous solutions obtained as process condensates in coupled ammonia and urea syntheses.

In the preparation of ammonia, starting from hydrocarbons, mostly methane, a synthesis is prepared first in a primary reformer by reaction of the hydrocarbon with excess steam, mainly according to the reaction equation of

$$CH_4+H_2O\rightarrow CO+H_2$$

Subsequently, air is mixed with the gas mixture leaving the primary reformer, in order to further convert, with oxygen, in a secondary reformer, any hydrocarbon present and, moreover, to introduce the nitrogen required for the ammonia synthesis. The gas mixture leaving the secondary reformer mainly consists of water vapour, carbon dioxide, nitrogen, carbon monoxide and hydrogen. This mixture is treated catalytically for the purpose of converting carbon monoxide, mainly according to the reaction equation of

$$CO+H_2O\rightarrow CO_2+H_2$$

so that a mixture of carbon dioxide, nitrogen, hydrogen, water vapour and small quantities of carbon monoxide is obtained. The excess water vapour in this mixture is condensed and forms the so-called process condensate, the carbon dioxide is removed by washing and the small quantities of carbon monoxide are converted, in a methanator, into the methane not poisonous to the ammonia synthesis catalyst, so that a suitable starting mixture for the ammonia synthesis results.

In the various processing steps small quantities of byproducts are formed as well, such as ammonia in the secondary reformer, methanol, methylamine and other organic impurities in the carbon monoxide conversion.

In the condensation of the excess water vapour these byproducts will enter the process condensate. The process condensate, moreover, contains dissolved carbon dioxide and traces of metal compounds originating from catalysts and equipment.

These impurities present in the process condensate are undesirable if the process condensate is to be fed back into the system as boilder feed water or if it is to be discharged as waste water.

The quantity of process condensate in an ammonia plant depends on the steam/hydrocarbon ratio in the primary reformer and, including the small quantities of condensate from the coolers of the synthesis gas compressor and condensate obtained in the methanator, amounts to about 1.1—1.25 tonnes per tonne ammonia produced. This condensate as a rule contains 0.08—0.1% by weight ammonia, 0.15—0.2% by weight carbon dioxide, 0.1—0.2% by weight methanol, 30—50 ppm organic impurities and traces of the metals iron, copper, zinc, aluminium, sodium and calcium.

In the preparation of urea from ammonia and carbon dioxide a urea synthesis solution still containing a substantial quantity of free ammonia and non-converted ammonium carbamate is formed at high temperature and the pressure belonging thereto. The carbamate is decomposed, in one or more steps, into ammonia and carbon dioxide, which are driven off for the larger part with the free ammonia present and usually recirculated. In the final decomposition step an aqueous urea solution is obtained still containing dissplved quantities of ammonia and carbon dioxide, which are subsequently removed by expansion to atmospheric or lower pressure. The aqueous urea solution is concentrated by evaporation and/or crystallization and further processed. During the evaporation process a gas mixture is formed containing, in addition to water vapour, entrained fine drops of urea and further also ammonia and carbon dioxide. This gas mixture, as well as the gas mixture separated off during the expansion of the urea solution after the final decomposition step, is condensed and the process condensate thus obtained is partly fed back into the process for absorbing the gas mixture carried off from the final decomposition step. The remaining part is, as a rule, discharged.

The process condensate also incorporates the water fed into the process as steam for operating the ejectors in the evaporation and/or crystallization section, washing water, flushing water to the stuffing boxes of the carbamate pumps, etc. Per mole urea a mole water is formed. So in a urea plant with a capacity of 1900 tonnes urea a day 570 tonnes water is formed per day. In addition, about 600 tonnes water a day is fed in, so that per day roughly 1170 tonnes water in all must be carried off.

The 2—9% by weight $NH_3$, 0.8—6% by weight $CO_2$ and 0.3—1.5% by weight urea present in this water on the one side represent substantial quantities of raw materials and product and would, on the other side, load the surface water into which this waste water would be discharged to an extent no longer permitted by the authorities in many countries.

Consequently, in coupled installations for the preparation of ammonia and urea large quantities of process condensate are formed in both installations, which quantities between them show great differences in composition and which can be discharged or be used as boiler feed water only after careful purification.

In the Netherlands patent application 7705356 it has already been suggested to form superheated steam from the process condensates obtained in the ammonia preparation and in the urea preparation. To this end the process condensate obtained in the urea preparation is fed into a hydrolyzing column and the urea is hydrolyzed. The ammonia and carbon dioxide formed in the

hydrolysis are desorbed in a desorption column and the remaining solution, together with the process condensate obtained in the ammonia preparation, is converted, optionally after removal of ammonia and carbon dioxide by expansion of the solutions put together and stripping of the gases liberated in this process, into superheated steam, which is fed to the primary reformer.

The disadvantage of the process described is that the hydrolysis of urea in the presence of ammonia in the neighbourhood of the equilibrium conditions results in higher final urea concentrations than in the absence of ammonia. The danger of an incomplete hydrolysis is therefore not imaginary. When urea-containing process condensate is converted into steam, the urea in the steam boiler will decompose into ammonia and carbon dioxide and constitute a danger of corrosion here.

Now, the purpose of the invention is to provide a process by means of which process condensates obtained in the ammonia and urea preparation can be processed together without formation of flows of waste affecting the environment, in which process the said disadvantage is avoided. To this end a urea-containing process condensate relatively poor in ammonia is fed into the upper part of a reaction column at such a temperature and pressure that urea is decomposed herein into ammonia and carbon dioxide and steam serving not only as heating agent but also as stripping agent is fed into the bottom of the column.

The invention consequently relates to a process for the removal of urea, ammonia and carbon dioxide from dilute aqueous solutions obtained as process condensates in coupled ammonia and urea syntheses by hydrolysis of urea and desorption of ammonia and carbon dioxide and is characterized in that process condensate poor in ammonia is treated with steam in a reaction column at a pressure of 1500—4200 kPa and a temperature of 200—320°C, a gas mixture containing ammonia, carbon dioxide and water vapour is carried off from the top of the reaction column and is added to the steam used for conversion of natural gas in the primary reformer of the synthesis gas preparation and an aqueous liquid virtually free of urea, ammonia and carbon dioxide is carried off from the bottom of the column.

Preferably pressures of 3200—4000 kPa and temperatures of 220—280°C are applied, because under these circumstances a virtually complete hydrolysis of the urea takes place in a short time.

The process condensate of the urea synthesis contains relatively much ammonia in respect of the process condensate of the ammonia synthesis. In order to be able to supply to the reaction column a process condensate poor in ammonia, a maximum quantity of ammonia and carbon dioxide is removed, by stripping in a desorption column at a pressure of 100—500 kPa, from the high-ammonia process condensate of the urea synthesis prior to the treatment in the reaction column. In this process, steam with a pressure of 100—500 kPa can be used as heating and stripping agent. The gas mixture obtained from the top of the desorption column is wholly or partly condensed. A part of the condensate is used as reflux. The main flow and any non-condensed part of the gas mixture is supplied to the low-pressure carbamate condenser of the urea synthesis. It is also possible to supply the gas mixture obtained from the top of the desorption column to the low-pressure carbamate condenser of the urea synthesis without condensation

In the desorption column, so much ammonia and carbon dioxide from the process condensate have to be removed that the flow fed to the reaction column contains less than 0.3% by weight $NH_3$.

The low-ammonia process condensates of the ammonia and the urea preparations can be fed to the reaction column both as separate flows and as one flow. It is also possible to partly hydrolyze in a separate heating zone a part of the urea present in the low-ammonia process condensate originating from the urea synthesis. Such a treatment can be effected by passing the low-ammonia process condensate carried off from the bottom of the desorption column through a heating zone before treatment in the reaction column. The heating zone can be formed, for instance, by a wide tube provided with means to supply heat. If so desired, the wide tube can be placed inside the reaction column in such a manner that the low-ammonia process condensate flows through an overflow into the top of the reaction column. The heating of the low-ammonia process condensate can then be effected by the heat of the steam fed into the reaction column. Another variant of the process according to the invention consists in a part of the mixture from the upper part of the reaction column being carried off to a heating zone and being fed back into the reaction column. It is true that in both variants an extra heating zone is required, but on the other hand the reaction column may be of smaller dimensions and the residence time in the column may be shorter.

The heating and stripping agent to be applied in the reaction column is preferably steam of 1500—4200 kPa. It is true that other inert gases can be used as well, but they must be separated off again, which involves extra costs.

The invention will subsequently be elucidated by means of the figures and be explained by means of the examples without, however, being limited thereto.

In applying the process according to Figure 1 the process condensate of the urea preparation, collected in tank 1, is brought to a pressure of 100—500 Pa by means of pump 2 and fed, via heat exchanger 3, into desorption column 4. Herein the larger part of the dissolved ammonia and carbon dioxide is driven out by the stripping action of low-pressure steam fed through 5 into the lower half and the heat present in this steam.

The gas mixture thus separated off through 6, which gas mixture also contains a quantity of water vapour, is condensed completely in reflux condenser 7. A small part of the condensate is returned to the top of desorption column 4, the larger part is supplied through a line 8 to the urea synthesis, for instance to the condensation and absorption zone of the final decomposition step.

Under a pressure of 1500—4200 kPa the solution treated in desorption column 4 and still containing, in addition to small quantities of ammonia and carbon dioxide, virtually the whole quantity of urea orignally present, is subsequently fed, by means of pump 9, into the top of reaction column 15 via heat exchanger 10 and a line 13.

The process condensate of the ammonia preparation, collected in tank 11, is brought to the said pressure by pump 12 and fed also via heat exchanger 10 and line 13 into the top of reaction column 15 together with the process condensate of the urea preparation. Reaction column 15 is divided by, for instance, perforated plates into a number of compartments serving as ideal sparged gas contractors. Into the bottom of this reaction column steam of 1500—4200 kPa is fed through 14. The temperature in the column is controlled by metering the quantity of steam and the pressure in the reaction column. The steam supplies the heat required for the decomposition of carbamate formed by hydrolysis of urea and the evaporation of the ammonia and carbon dioxide formed in this process. The urea content of the solution flowing down in reaction column 15 falls fairly rapidly, and that more rapidly as the temperature is higher. The final urea content is found, after a certain residence time, to be determined by the concentrations of ammonia and carbon dioxide in the liquid. For this reason the pre-desorption of ammonia and carbon dioxide is often necessary to reach the required low urea content of 10 ppm or lower. At an average temperature of 245°C, for instance, a minimum residence time in the reaction column of about 5—10 minutes is required. As the average temperature in the reaction column is higher a shorter residence time will suffice.

The mixture of gases driven off and steam, formed in reaction column 15, is carried off from the top and fed, through 16, to a non-drawn primary reformer for the preparation of synthesis gas. The ammonia, methanol and other organic components present in the gas mixture are broken down in the primary reformer. From the bottom of reaction column 15 a liquid flow is carried off with urea and ammonia concentrations each of which may be 10 ppm or lower, depending on the quantity of steam. In heat exchanger 10 this liquid flow gives off heat to the feed to reaction column 15 and is subsequently fed, through 17, to a non-drawn device for the removal of metal compounds and for the preparation of boiler feed water, or discharged wholly or partly.

In applying the process according to Figure 2 the process condensate of the urea synthesis is passed, after removal in desorption column 4 of as much ammonia and carbon dioxide as possible, through a heating zone 18 prior to the treatment in the reaction column in order here to hydrolyze already a part of the urea. The low-ammonia process condensate is brought by pump 9 under a pressure of 1500—4200 kPa bar and fed via heat exchanger 10 to heating zone 18. At the same time a part of the steam supplied through line 14 is fed to it through 14a. From the top of heating zone 18 the vapour-liquid mixture formed is fed, through 19, into reaction column 15, to which is fed, through 14b, the rest of the steam required for the decomposition of urea and for driving off the ammonia and carbon dioxide formed in this process. The process condensate of the ammonia preparation is fed separately, via pump 12, heat exchanger 10 and line 13, into the top of reaction column 15.

Example 1

By means of the process according to figure 1 process condensate was treated which had been obtained in a urea plant with an output of 1900 tonnes a day and in the ammonia plant coupled to it, having an output of 1130 tonnes a day.

The quantities are given in kg/hour.

The process condensate of the urea plant, 48345 kg, contained 778 kg urea, 1554 kg $NH_3$, 878 kg $CO_2$ and 45135 kg water, was heated in heat exchanger 3 from 40°C to 80°C. In desorption column 4 this solution was made to flow, at a pressure of 400 kPa, countercurrently to 9800 kg steam with a temperature of 143°C and a pressure of 450 kPa. From the top of desorption column 4 a gas mixture was carried off the composition of which was 2008 kg $NH_3$, 1114 kg $CO_2$, 2102 kg $H_2O$. This gas mixture was condensed completely, and of the solution thus obtained, which had a temperature of 45°C, a part, consisting of 463 kg $NH_3$, 263 kg $CO_2$ and 485 kg $H_2O$, was returned as reflux into the desorption column. The remaining part was returned to the urea plant. The bottom product of desorption column 4 contained, in addition to 53318 kg $H_2O$, also 9 kg $NH_3$ and 778 kg urea and had a temperature of 100°C. This solution was brought to a pressure of 3800 kPa by means of pump 9.

The process condensate of the ammonia plant, 52400 kg, contained 60 kg $NH_3$, 190 kg $CO_2$, 110 kg $CH_3OH$ and 52040 kg $H_2O$, had a temperature of 50°C and was brought under a pressure of 3800 kPa by means of pump 12 and heated, together with the said process condensate of the urea plant, to 240°C in heat exchanger 10 and fed into the top of reaction column 15. The quantity of steam fed into the bottom of this reaction column was 40.000 kg, its temperature was 352°C and the pressure 3800 kPa. The flow rate was regulated so that the residence time of the liquid was 5—10 minutes. The urea present in the feed was virtually completely hydrolyzed to form $NH_3$ and $CO_2$. From the top of the reaction column 30620 kg gas mixture was carried off, the temperature of

which was 242°C and the pressure 3700 kPa. The gas mixture, which contained 95.5% $H_2O$, 1.6% $NH_3$, 2.5% $CO_2$ and 0.35% $CH_3OH$, was fed to the primary reformer. The bottom product from the reaction column consisted of 115885 kg water, in which 10 ppm $NH_3$, 5 ppm urea, 25 ppm methanol and traces of heavy metals, and was used for pre-heating the feed to the reaction column, in which process the temperature fell from 246°C to 106°C. This was subsequently fed through 17 to the boiler feed water system.

Example 2

By means of the process as represented in figure 2 the same quantities of process condensate of the urea plant and the ammonia plant as described in Example 1 were processed. The treatment of the process condensate of the urea plant in desorption column 4 was carried out in the same way as described in Example 1. Via pump 9 54105 kg solution was fed, under a pressure of 3800 kPa and at a temperature of 100°C, into heating zone 18. The composition of the solution was: 53318 kg $H_2O$, 9 kg $NH_3$ and 778 kg urea. Into this heating zone 12.000 kg steam with a temperature of 352°C and a pressure of 3800 kPa was fed through 14a. From the top of the heating zone 66105 kg liquid-vapour-mixture was carried off, the composition of which was: 65108 kg $H_2O$, 406 kg $NH_3$, 513 kg $CO_2$, 78 kg urea. The flow rate was regulated so that the residence time in the heating zone was about 8 minutes. The said liquid-vapour mixture was fed into the reaction column through line 19 and contacted with 28.000 kg steam. This steam, the temperature of which was 352°C and the pressure 3800 kPa, was supplied through 14b. The quantities of vapour and liquid carried off from the reaction column and their compositions correspond with those described in Example 1.

**Claims**

1. Process for the removal of urea, ammonia and carbon dioxide from dilute aqueous solutions obtained as process condensates in coupled ammonia and urea syntheses by hydrolysis of urea and desorption of ammonia and carbon dioxide, characterized in that process condensate containing less than 0,3% by weight of ammonia is treated with steam in a reaction column at a pressure of 1500—4200 kPa and a temperature of 200—320°C, a gas mixture containing ammonia, carbon dioxide and water is carried off from the top of the reaction column and is added to the steam used for the conversion of natural gas in the primary reformer of the synthesis gas preparation and an aqueous liquid virtually free of urea, ammonia and carbon dioxide is carried off from the bottom of the reaction column.

2. Process according to claim 1, characterized in that the pressure is 3200—4000 kPa and the temperature 220—280°C.

3. Process according to claim 1 or 2, characterized in that, prior to the treatment in the reaction column, ammonia and carbon dioxide are removed, at a pressure of 100—500 kPa, from the process condensate of the urea synthesis containing urea, ammonia and carbon dioxide.

4. Process according to claims 1—3, characterized in that, prior to the treatment in the reaction column, a part of the urea present in the low-ammonia process condensate of the urea synthesis is hydrolyzed in a separate heating step.

5. Process according to claim 4, characterized in that the separate heating step is carried out within the reaction column.

6. Process according to claims 1—3, characterized in that a part of the liquid from the reaction column is passed from the upper part of the column through a separate heating zone and is returned again to the reaction column.

7. Process according to claims 1—6, characterized in that in the reaction column steam with a pressure of 1500—4200 kPa bar is used.

8. Process according to claims 1—7, characterized in that the aqueous liquid carried off from the bottom of the reaction column is fed to the boilder feed water preparation system.

9. Process according to claims 1—3, characterized in that the solution to be fed to the reaction column is pre-heated by heat èxchange with the solution carried off from the bottom of the reaction column.

10. Process according to claim 3, characterized in that the gas mixture carried off from the desorption column is fed to the low pressure carbamate condenser of the urea synthesis.

11. Process according to claim 3, characterized in that the gas mixture carried off from the desorption column is wholly or partly condensed, a part of the dilute aqueous solution of ammonia and carbon dioxide obtained is returned as reflux and the remaining part of the solution, as well as any non-condensed part of the gas mixture, is fed to the low pressure carbamate condenser of the urea synthesis.

**Revendications**

1. Procédé pour la récupération de l'urée, de l'ammoniac et du dioxyde de carbone depuis des solutions aqueuses diluées obtenues comme condensats de traitement dans les synthèses couplées de l'ammoniac et de l'urée par hydrolyse de l'urée et désorption de l'ammoniac et du dioxyde de carbone, caractérisé en ce que le condensat de traitement contenant moins de 0,3% en poids d'ammoniac est traité avec de la vapeur d'eau dans une colonne de réaction sous une pression de 1500—4200 kPa et à une température de 200—320°C, qu'un mélange gazeux contenant de l'ammoniac, du dioxyde de carbone et de l'eau est évacué du haut de la colonne de réaction et est ajouté à la vapeur d'eau utilisée pour la transformation du gaz naturel dans le reformeur primaire de la préparation du gaz de synthèse et qu'un liquide aqueux pratiquement exempt d'urée, d'ammoniac et de dioxyde

de carbone est évacué du bas de la colonne de réaction.

2. Procédé selon la revendication 1, caracterisé en ce que la pression est de 3200—4000 kPa et que la température est de 220—280°C.

3. Procédé selon la revendication 1 ou 2, caractérisé en ce que, avant le traitement dans la colonne de réaction, l'ammoniac et le dioxyde de carbone sont éliminés, sous une pression de 100—500 kPa, du condensat de traitement de la synthèse de l'urée contenant de l'urée, de l'ammoniac et du dioxyde de carbone.

4. Procédé selon les revendications 1 à 3, caractérisé en ce que avant le traitement dans la colonne de réaction, une partie de l'urée présente dans le condensat de traitement pauvre en ammoniac de la synthèse de l'urée est hydrolysée dans une étape de chauffage séparée.

5. Procédé selon la revendication 4, caractérisé en ce que l'étape de chauffage séparée est réalisée dans la colonne de réaction.

6. Procédé selon les revendications 1 à 3, caractérisé en ce que l'on fait passer une partie du liquide provenant de la colonne de réaction, depuis la partie supérieure de la colonne, à travers une zone de chauffage séparée et qu'on la renvoie dans la colonne de réaction.

7. Procédé selon les revendications 1 à 6, caractérisé en ce que l'on utilise de la vapeur d'eau dans la colonne de réaction sous une pression de 1500—4200 kPa.

8. Procédé selon les revendications 1 à 7, caractérisé en ce que le liquide aqueux évacué du bas de la colonne de réaction est chargé dans le système de préparation de l'eau d'alimentation du bouilleur.

9. Procédé selon les revendications 1 à 8, caractérisé en ce que la solution à introduire dans la colonne de réaction est préchauffée par échange de chaleur avec la solution évacuée du bas de la colonne de réaction.

10. Procédé selon la revendication 3, caractérisé en ce que le mélange gazeux évacué de la colonne de désorption est chargé dans le condenseur de carbamate à basse pression de la synthèse de l'urée.

11. Procédé selon la revendication 3, caractérisé en ce que le mélange gazeux évacué de la colonne de désorption est condensé tout ou partie, qu'une partie de la solution aqueuse diluée d'ammoniac et de dioxyde de carbone obtenue est recyclée comme reflux et que la partie restante de la solution, ainsi que toute partie non condensée du mélange gazeux, est chargée dans le condenseur de carbamate à basse pression de la synthèse de l'urée.

**Patentansprüche**

1. Verfahren zur Entfernung von Harnstoff, Ammoniak und Kohlendioxid aus verdünnten wässerigen Lösungen, die als Prozeßkondensate in gekoppelten Ammoniak- und Harnstoffsynthesen erhalten werden, durch Hydrolyse von Harnstoff und Desorption von Ammoniak und Kohlendioxid, dadurch gekennzeichnet, daß man Prozeßkondensat enthaltend weniger als 0,3 Massen-% Ammoniak mit Wasserdampf in einer Reaktionskolonne bei einem Druck von 1500—4200 kPa und einer Temperature von 200—230°C behandelt, ein Gasgemisch enthaltend Ammoniak, Kohlendioxid und Wasser vom Kopf der Reaktionskolonne abzieht und dem Wasserdampf der zur Konvertierung des Erdgases im Primärreformer der Synthesegasherstellung verwendet wird, zusetzt, und eine im wesentlichen von Harnstoff, Ammoniak und Kohlendioxid freie wässerige Flüssigkeit vom Boden der Reaktionskolonne abzieht.

2. Verfahren nach Anspruch 1, dadurch gekennzeichnet, daß der Druck 3200 bis 4000 kPa und die Temperatur 220—280°C beträgt.

3. Verfahren nach Anspruch 1 oder 2, dadurch gekennzeichnet, daß vor der Behandlung in der Reaktionskolonne Ammoniak und Kohlendioxid bei einem Druck von 100—500 kPa aus dem Prozeßkondensat der Harnstoffsynthese, das Harnstoff, Ammoniak und Kohlendioxid enthält, entfernt werden.

4. Verfahren nach den Ansprüchen 1—3, dadurch gekennzeichnet, daß vor der Behandlung in der Reaktionskolonne ein Teil des im Prozeßkondensat mit niedrigem Ammoniakgehalt der Harnstoffsynthese zugegenen Harnstoffes in einer separaten Erwärmungsstufe hydrolysiert wird.

5. Verfahren nach Anspruch 4, dadurch gekennzeichnet, daß die separate Erwärmungsstufe innerhalb der Reaktionskolonne durchgeführt wird.

6. Verfahren nach den Ansprüchen 1—3, dadurch gekennzeichnet, daß ein Teil der Flüssigkeit aus der Reaktionskolonne vom oberen Teil der Kolonne durch eine separate Erwärmungszone geführt und wieder in die Reaktionskolonne rückgeführt wird.

7. Verfahren nach den Ansprüchen 1—6, dadurch gekennzeichnet, daß in der Reaktionskolonne ein Dampf mit einem Druck von 1500—4200 kPa eingesetzt wird.

8. Verfahren nach den Ansprüchen 1—7, dadurch gekennzeichnet, daß die vom Boden der Reaktionskolonne abgezogene wässerige Flüssigkeit dem Kesselspeisewasser-Aufbereitungssystem zugeführt wird.

9. Verfahren nach den Ansprüchen 1—3, dadurch gekennzeichnet, daß die der Reaktionskolonne zuzuführende Lösung durch Wärmeaustausch mit dem vom Boden der Reaktionskolonne abgezogenen Lösung vorgewärmt wird.

10. Verfahren nach Anspruch 3, dadurch gekennzeichnet, daß das aus der Desorptionskolonne abgezogene Gasgemisch dem Niederdruck-Carbamatkondensator der Harnstoffsynthese zugeführt wird.

11. Verfahren nach Anspruch 3, dadurch gekennzeichnet, daß das aus der Desorptionskolonne abgezogene Gasgemisch zur Gänze oder teilweise kondensiert wird, ein Teil der erhaltenen

verdünnten wässerigen Lösung von Ammoniak und Kohlendioxid als Rückfluß rückgeführt wird und der verbleibende als Rückfluß rückgeführt wird und der verbleibende Teil der Lösung sowie jedweder nichtkondensierte Anteil des Gasgemisches dem Niederdruck-Carbamatkondensator der Harnstoffsynthese zugeführt werden.

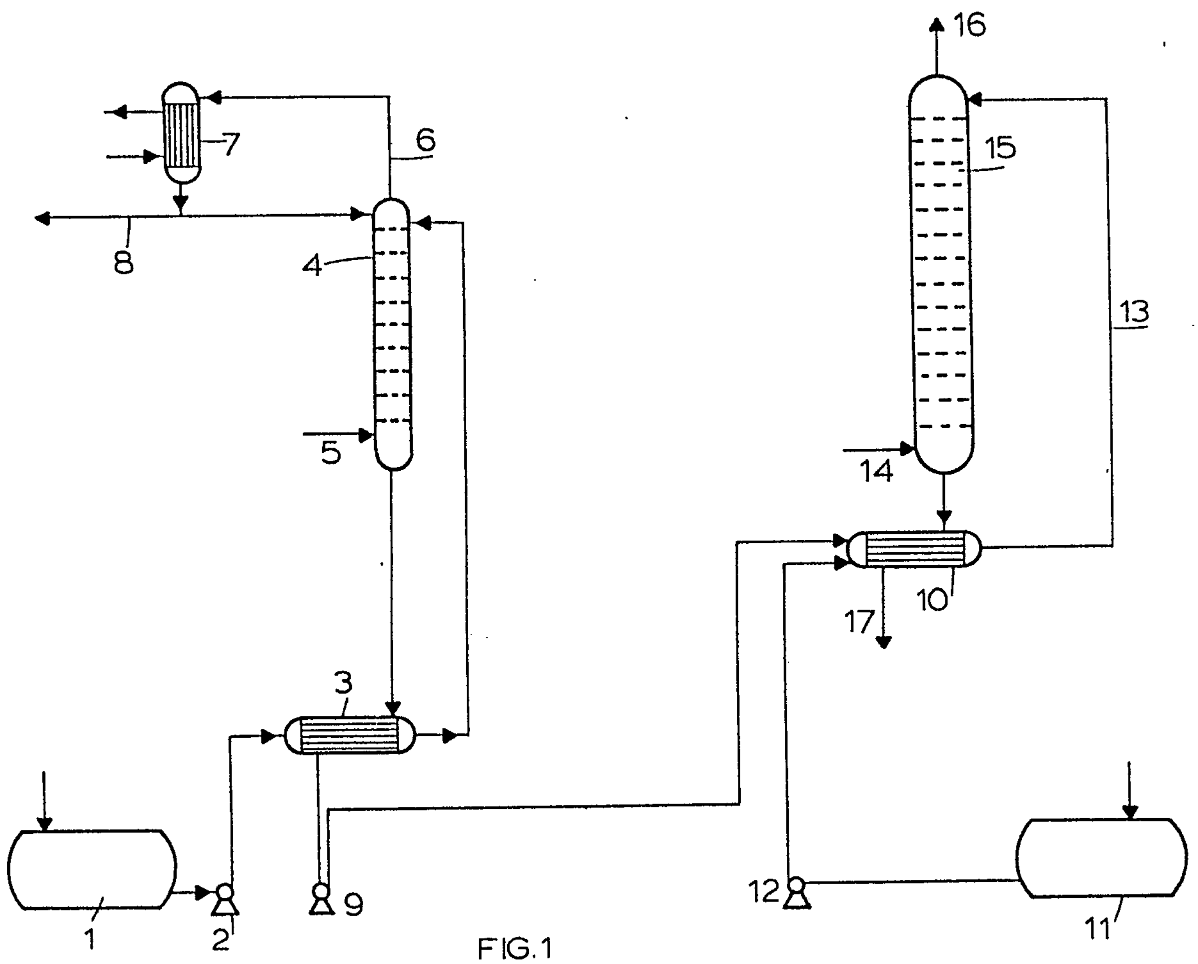


FIG.1

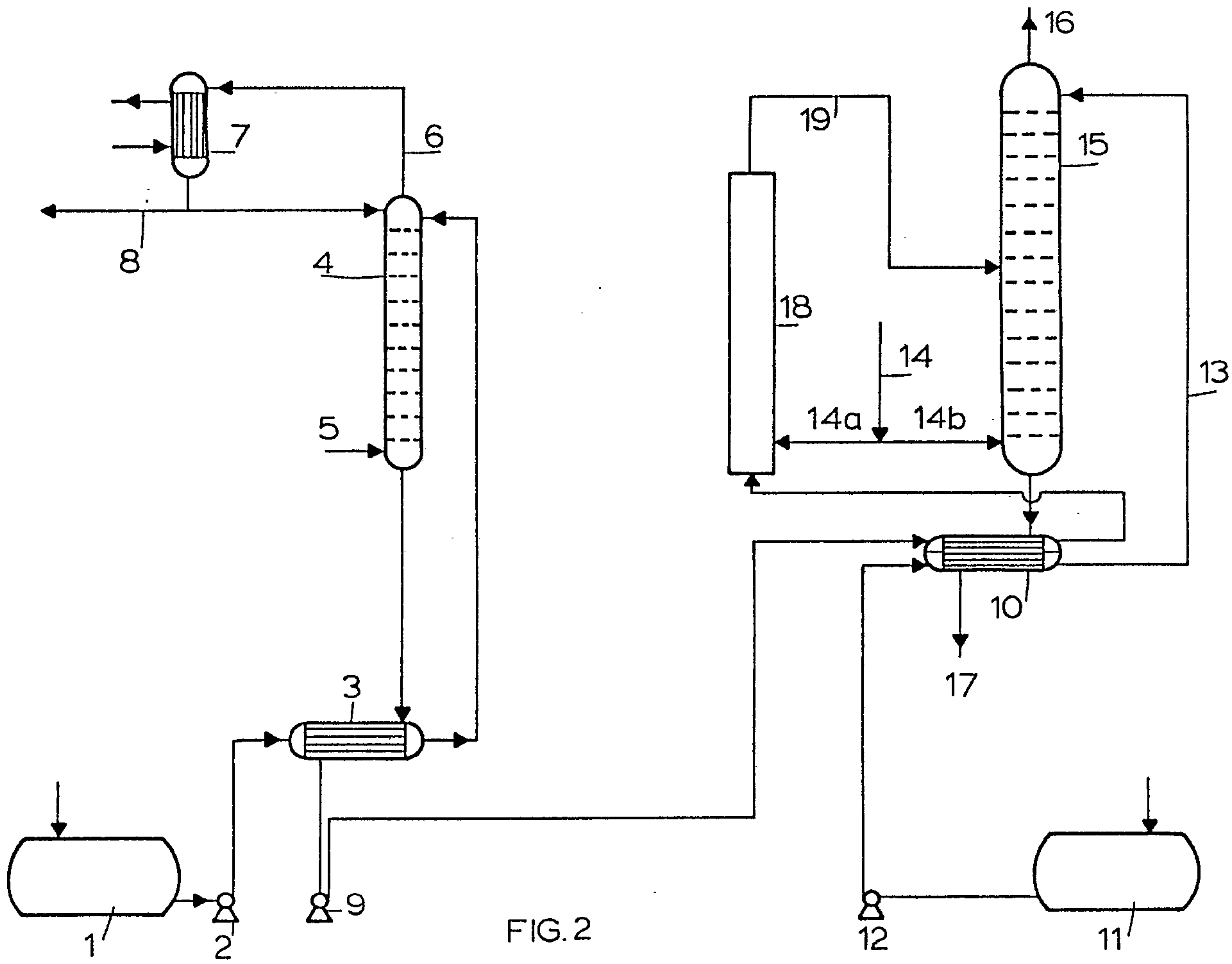


FIG. 2